# EUROPÄISCHE PATENTSCHRIFT

(11) **EP 0 436 015 B1**
(45) Veröffentlichungstag und Bekanntmachung des Hinweises auf die Patenterteilung: **21.09.1994**
(21) Anmeldenummer: 90913192.2
(22) Anmeldetag: 20.07.1990
(51) Int. Cl.: A61K 9/02

(54) **PHARMAZEUTISCHE ZUBEREITUNG ZUR REKTALEN VERABREICHUNG**
RECTALLY ADMINISTERED PHARMACEUTICAL PREPARATION
PREPARATION PHARMACEUTIQUE POUR ADMINISTRATION RECTALE

(30) Priorität: 25.07.1989 DE 3924570
(43) Veröffentlichungstag der Anmeldung: 10.07.1991
(73) Patentinhaber: Henning Berlin Anlagen GmbH, D-12099 Berlin (DE)
(72) Erfinder: LAHR, Wolfgang, D-1000 Berlin 33 (DE)
(74) Vertreter: Andrae, Steffen, Dr.
(86) Internationale Anmeldenummer: EP9001204
(87) Internationale Veröffentlichungsnummer: WO9101129

(56) Entgegenhaltungen:
- EP-A- 0 050 981
- EP-A- 0 062 000
- DE-A- 3 106 619
- FR-A- 2 381 520
- US-A- 4 664 256
- Lebensmittel-Lexikon, 2. Auflage, VEB Fachbuchverlag Leipzig (1981), S.875
- Lexikon der Pharmazie, S.Ebel u.H.J.Roth, Georg Thieme Verlag Stuttgart-New York, 1987, S.128

## Beschreibung

Die Erfindung betrifft pharmazeutische Zubereitungen zur rektalen Verabreichung, die in Form einer wäßrigen Lösung, Emulsion oder Suspension eines Wirkstoffs oder Wirkstoffgemischs, daß heißt als Klistiere (Klysmen),verabreicht werden. Die vorliegende Erfindung betrifft somit eine neue Arzneiform, die zur Therapie sowohl im Human- als auch Veterinärbereich anwendbar ist.

Der einschlägige Stand der Technik ist durch handelsübliche Klysmen gekennzeichnet, die als sogenannte Fertigklysmen bereitgestellt werden, also bereits die flüssige Form darstellen.

Der markanteste Nachteil der Fertigklysmen ist, daß sie ein fixes Volumen darstellen. Dieses Volumen muß vollständig appliziert werden, um die gesamte Wirkstoffdosis über möglichst lange Zeit zur Wirkung bringen zu können. Dies bereitet vielen Patienten aus anatomischen und/oder pathologischen Gründen Schwierigkeiten. Bei eingeschränkter Rückhaltefähigkeit des Patienten wird die Arzneiform rasch ganz oder teilweise wieder ausgeschieden.

Fertigklysmen enthalten als Flüssigkeitsphase aus pharmakologischen Gründen überwiegend Wasser. Wäßrige Systeme sind jedoch anfällig gegen mikrobiellen Befall bei Herstellung, Abfüllung und Lagerung. Entsprechend müssen Konservierungsstoffe zugesetzt werden. Diese wiederum besitzen eine hohe allergene Potenz, sind selbst in wässrigen Lösungen instabil (z.B. sind die am häufigsten verwendeten Benzoesäureester hydrolyseempfindlich) und werden von Kunststoffen, aus denen Klysmenflaschen bestehen, absorbiert (vergl. Wallhäußer, Praxis der Sterilisation, Thieme-Verlag, 1984, S. 333, 380).

Fertigklysmen enthalten weiterhin häufig Stabilisatoren zur chemischen Stabilisierung der Wirkstoffe gegen Hydrolyse, Oxidation und/oder andere Umlagerungsreaktionen sowie Anticakingzusätze, um bei Suspensionen eine Sedimentation der Inhaltsstoffe zu vermeiden bzw. eine Resuspendierung nach Sedimentation zu gewährleisten. Wird auf Stabilisatoren bzw. Konservantien verzichtet, so resultieren daraus für derartige Arzneimittel besondere Lagerungshinweise (z.B. "im Kühlschrank lagern") und eine beschränkte Verkehrsfähigkeit (Verfalldatum).

Im kanadischen Patent 1 230 056 werden als repräsentativer Stand der Technik 5-Aminosalicylsäure-Klysmen beschrieben, die unter Verwendung von Komplexbildnern, Antioxidantien und unter Inertbegasung als Lösung oder Suspension hergestellt werden.

Mulder et al, Scand. J. of Gastroentero., 1988, 23, S. 1006 beschreibt Klysmen, bestehend aus 5-Aminosalicylsäure und Prednisolonphosphat-Natriumsalz sowie der doppelten Menge Methylhydroxybenzoat als Konservierungsmittel, bezogen auf den Wirkstoff Prednisolonphosphat, wobei die Stabilität derartiger Klysmen selbst bei 4°C nur über 4 Wochen gewährleistet war. Bondesen et al., Scand. J. of Gastroentero., 1984, Vol. 19, Nr. 5, S. 197 beschreibt 5-Aminosalicylsäure enthaltende Klysmen in denen Komplexbildner, Antioxidantien bzw. Konservantien verwendet werden. In Lancet vom 13.3.1982, S. 579 wird die technische Lehre vermittelt, Beclometasondipropionat-Fertigklysmen unter Verwendung der Konservantien Propylhydroxybenzoat und Ethylhydroxybenzoat herzustellen. Alle diese aus dem Stand der Technik bekannten Klysmen weisen die eingangs geschilderten Nachteile sowie den für Fertigklysmen charakteristischen ökonomischen Nachteil auf, daß diese ein hohes Gewicht, bezogen auf den wirksamen Bestandteil, aufweisen, was zu hohen Transportkosten führt.

EP-A-0 062 000 beschreibt eine feste Vorläuferzubereitung für ein Klysma, die als Wirkstoff 4-Aminosalicylsäure und als Hilfsstoffe Laktose, das Fließmittel kolloidales SiO₂ und die ionogenen Verdickungsmittel Natriumcarboxymethylcellulose und Natriumstärkeglykolsäureester enthält und vor der Verabreichung mit Wasser versetzt werden soll.

FR-A-2 381 520 beschreibt micellare Lösungen zur rektalen Verabreichung, die durch Trocknen in Pulver überführt werden können und auch in dieser Form zur Herstellung von Zubereitungen für die rektale Verabreichung verwendet werden können.

Es ist Aufgabe der vorliegenden Erfindung, pharmazeutische Zubereitungen zur rektalen Verabreichung in Form einer wäßrigen Lösung, Emulsion oder Suspension eines Wirkstoffs oder Wirkstoffgemischs so auszugestalten, daß eine patientenindividuelle Volumendosierung bei gleichzeitiger Verabreichung der vollständigen Wirkstoffdosis ermöglicht wird und eine verbesserte Lagerstabilität erhalten wird, ohne daß auf Konservantien und Stabilisierungsmittel zurückgegriffen werden muß.

Diese Aufgabe wird bei einer pharmazeutischen Zubereitung gemäß Oberbegriff von Patentanspruch 1 dadurch gelöst, daß sie die Merkmale gemäß Kennzeichen des Patentanspruchs 1 aufweist.

Vorteilhafte Ausgestaltungen einer solchen Zubereitung sowie vorteilhafte Verwendungen sind den Unteransprüchen zu entnehmen.

Die vorliegende Erfindung beruht auf der Feststellung, daß es möglich ist, trockene, konservierungsmittelfreie Klistierzubereitung zu formulieren, die "kaltwasserlöslich sind", was im Rahmen der vorliegenden Anmeldung bedeuten soll, daß sie erst unmittelbar vor ihrer Anwendung durch Zugabe von Wasser von Raum- oder Körpertemperatur rekonstituiert werden, d.h. in eine applizierbare Form als Lösung, Emulsion oder Suspension überführt werden können. Indem derartige feste ("trockene") Zubereitungen in fester Form in Klysmenflaschen mit geeigneten Skalen hergestellt und in den Handel gebracht werden, verbessert sich die Lagerstabilität, vermindert sich das Transportgewicht und ist es insbesondere möglich, die Trockenzubereitung in variablen, reproduzierbaren Volumina aufzulösen oder zu dispergieren, die den Therapieerfordernissen individuell gerecht werden.

Die erfindungsgemäßen Zubereitungen enthalten nebeneinander Wirkstoffe bzw. Wirkstoffgemische und Hilfsstoffe, die eine Auflösung oder Dispergierung der Wirkstoffe in zugesetztem Wasser ermöglichen. Aufgrund der unterschiedlichen Natur verwendbarer Wirkstoffe können die Wirkstoffgehalte bei den erfindungsgemäßen Zubereitungen in sehr weiten Grenzen variieren, wobei ein Bereich von 0,01 bis 95 Gew.-% den für die praktische Anwendung in der Regel möglichen Bereich der Wirkstoffmengen erfassen sollte. Der Wirkstoffgehalt einer spezifischen trockenen Klysmazubereitung hängt sehr entscheidend von dem Typ und Charakter des verwendeten Wirkstoffs und der für diesen Wirkstoff typischen therapeutisch erforderlichen Dosis ab. Die Hilfsstoffzusammensetzungen sind in der Regel in quantitativer und qualitativer Hinsicht auf den spezifischen Wirkstoff abgestimmt und bilden in der Regel von 99,99 bis 5 Gew.-% der Zubereitung. Es ist aber darauf hinzuweisen, daß sich Wirkstoffmengen und Hilfsstoffmengen nicht auf 100 Gew.-% ergänzen müssen, da die festen Zubereitungen beispielsweise auch noch gewisse Feuchtigkeitsmengen enthalten können. Was den Einfluß der Art der Wirkstoffe angeht, so werden Hormone beispielsweise im Mikrogramm-Bereich, Corticoide im Milligramm-Bereich und Arzneimittel wie Mesalazin in Mengen bis zu mehreren Gramm pro Einzeldosis verabreicht. Daraus ergeben sich die geschilderten weiten Grenzen für den Wirkstoffgehalt und entsprechend auch für den Wirkstoffanteil.

Die Rekonstitution der erfindungsgemäßen Trockenklysmen erfolgt vorzugsweise nach Zugabe von mehr als 5 ml "kaltem" Wasser durch Schütteln. Dabei würde in der Regel ein störender Schaum entstehen, dessen Bildung gemäß der vorliegenden Erfindung jedoch dadurch verhindert wird, daß die Zubereitung als einen Hilfsstoff ein Antischaummittel, in einer Menge von 0,1 bis 10 Gew.%, enthält. Dabei werden vorzugsweise wasserfreie Antischaummittel, besonders bevorzugt solch auf Siliconbasis, verwendet, die den Schaum zumraschen Zusammenbrechen bringen.

Ferner enthalten die erfindungsgemäßen Trockenklysmenzubereitungen ferner neben dem oder den Wirkstoffen Gelbildner (Verdicker), die bei Raumtemperatur oder Körpertemperatur in Wasser eine Gelstruktur ausbilden, beispielsweise Gelbildner in Form von Cellulosederivaten, und zwar unabhängig davon, ob es sich bei dem rekonstituierten Klysma um eine Lösung oder eine Suspension handelt. Die erfindungsgemäßen Zubereitungen können derartige Gelbildner, insbesondere, wenn sie die Gelstruktur bevorzugt bei Körpertemperatur ausbilden, in deutlich höheren Mengen enthalten als die bisher üblichen Flüssigzubereitungen.

Weiterhin enthalten bevorzugte Ausführungsformen der erfindungsgemäßen Zubereitungen noch Netzmittel, um zum einen nach Zugabe von Wasser zu den trockenen Zubereitungen eine rasche Benetzbarkeit der Bestandteile zu gewährleisten, zum anderen, um eine gute Spreitfähigkeit im Darm zu erreichen.

Den jeweiligen Gegebenenheiten angepaßt, können die trockenen Zubereitungen weitere Hilfsstoffe zur Herstellung fester Lösungen oder als Granulierhilfsmittel oder aus anderen Gründen enthalten. Derartige Hilfsstoffe sind vorzugsweise Homo- oder Copolymere von Vinylpyrrolidon, Vinylacetat oder Vinylalkohol, Polyethylenglycole, insbesondere feste Polyethylenglycole mit mittleren Molekulargewichten bis zu 35 000 oder Mischungen davon, sowie andere oder weitere Hydrokolloide und Netzmittel, Fließverbesserer wie hochdisperses Siliciumdioxid, feste wasserlösliche Säuren, Puffersubstanzen und Zusätze zur Einstellung auf isotonische und physiologisch osmotische Verhältnisse wie Kochsalz, trockene Verdünnungsmittel wie Stärke, modifizierte Stärken, mikrokristalline Cellulose, Dextrine, Lactose, Zucker und auch Emulgatoren. Die Zubereitungen können als Bestandteile der festen Zubereitungen auch gewisse Anteile schwerflüchtiger flüssiger Bestandteile wie Glycerin, Propylenglykol, Polyethylenglycole mit mittleren Molekulargewichten bis zu 600 und Mischungen aus derartigen Hilfsstoffen enthalten.

Die Herstellung der erfindungsgemäßen trockenen Klysmenzubereitungen kann nach verschiedenen Verfahren erfolgen. Diese Verfahren sollen dabei Produkte liefern, die vorzugsweise praktisch wasserfrei sind, jedoch auf jeden Fall weniger als 15 Gew.-% Restfeuchte, vorzugsweise weniger als 10 Gew.-%, besonders bevorzugt weniger als 2 Gew.-% Restfeuchte, jeweils bezogen auf das Gesamtgewicht der trockenen Zubereitungen, enthalten. Chemisch gebundenes Wasser (Kristallwasser) ist dabei nicht berücksichtigt.

Die trockenen Klysmenzubereitungen können dabei nach an sich bekannten Methoden durch Mischen, Granulieren, Vermahlen, Instantisieren, Lyophylisieren, Kompaktieren, Verpressen, Sprühtrocknung, Sprüherstarrung oder kombinierten Verfahren hergestellt werden, wobei die Struktur der trockenen Zubereitung und ihre Zusammensetzung so ausgebildet sein muß, daß die Rekonstitution unter Zugabe von Wasser in möglichst kurzer Zeit, vorzugsweise in weniger als 10 min abgeschlossen ist. Zur Rekonstitution der anwendungsfertigen Zubereitung wird zu der Trockenzubereitung das gewünschte Volumen Wasser zugegeßen und mehrmals umgeschüttelt. Beschleunigt wird die Rekonstitution, wenn die Partikel der erfindungsgemäßen Zubereitungen möglichst feinteilig sind, wobei eine Größe von <1 mm, vorzugsweise von weniger als 500 µm bevorzugt ist.

Die erfindungsgemäßen Klysmenzubereitungen lassen sich prinzipiell mit allen Wirkstoffen herstellen, für die eine rektale Anwendung zur lokalen oder auch systemischen Wirkung angezeigt ist. Insbesondere bei Erkrankungen der unteren Darmabschnitte (Rectum und Colon), beispielsweise um Colitis Ulcerosa, Morbus Crohn, Proctitiden, Hämorrhoidalerkrankungen zu behandeln, aber auch zur Darmsterilisation lassen sie sich mit Erfolg verwenden. Als Wirkstoffe eignen sich Glucocorticoide, z.B. Betamethason, Beclometason, Hydrocortison, Prednisolon, Oxipurinol, daneben aber auch Aminosalicylsäurederivate wie beispielsweise 5-Amino-salicylsäure, Sulfasalazin. Antirheumatika, Analgetika, Sulfonamide, Antibiotika, Calciumantagonisten, Corticoide, Sedativa, Muskelrelaxantien, Migränemittel, Spasmolytika oder Lokalanästhetika stellen weitere Wirkstoffgruppen dar, die in die erfindungsgemäßen Zubereitungen eingearbeitet werden können, wobei die Wirkstoffe in Form ihrer freien Säuren, Basen, Salze, Ester, Hydrate und als Derivate davon vorliegen können. Sofern es therapeutisch erforderlich ist, können selbstverständlich auch Kombinationen von Wirkstoffen, insbesondere der genannten Wirkstoffe, verwendet werden.

Es hat sich gezeigt, daß bei den erfindungsgemäßen Zubereitungen auf Konservierungsmittel verzichtet werden kann. Auch sind Stabilisatoren sowie Anticaking-Zusätze verzichtbar, da die Zubereitungen alsbald nach ihrer Rekonstitution verbraucht werden. Besondere Lagerhinweise entfallen, die Verkehrsfähigkeit der Arzneiformen entspricht denen konventioneller fester Darreichungsformen und wird im wesentlichen durch die Stabilität des Wirkstoffs an sich im trockenen Zustand beeinflußt. Die Zeit zur Rekonstitution beträgt in der Regel weniger als 1 min bis zu 10 min. Danach sind die erfindungsgemäßen Formen fertig zur Anwendung.

Nachfolgend wird die Erfindung anhand vorteilhafter Ausführungsformen beispielhaft erläutert.

### Beispiel 1

0,44 g Betamethason-21-phosphat-Natriumsalz werden bei 70° C in 65,56 g geschmolzenen Polyethylenglykol 6000 klar gelöst. Der Schmelze werden 33 g Hydroxypropylmethylcellulose (Methocel^{(R)}E5) sowie 1.0 g wasserfreies Silicon-Antischaummittel zugegeben. Die Schmelze wird entweder durch Ausgießen zu ca. 1 cm dicken Schichten oder Sprüherstarrung zum Erkalten gebracht.

Im ersteren Fall wird die erstarrte Schmelze durch Vermahlen auf eine Korngröße < 350 µm gebracht. Je 1,5 g der erstarrten Schmelze, entsprechend 6,6 mg Betamethason-21-phosphat-Natriumsalz, werden in Klysmenflaschen mit einer Graduierung von 10 bis 100 ml gegeben. In einer Versuchsreihe werden abgestuft 10 ml, 20 ml, 50 ml, 100 ml handwarmes Wasser eingefüllt. Nach mehrmaligem kräftigen Umschütteln entstand innerhalb von 30 - 60 Sekunden eine fast klare, schwach opalisierende Lösung. Der beim Schütteln entstandene Schaum brach sofort zusammen.

### Beispiel 2

Analog Bsp. 1 wurde eine Trockenklysmazubereitung aus 6,0 g Beclometasondipropionat, 1988 g Polyethylenglykol 6000, 1000 g Hydroxypropylmethylcellulose und 6,0 g wasserfreiem Silikon-Antischaummittel hergestellt. Dosen von 1,5 g erstarrter Schmelze wurden mit 10, 20, 50, 100 ml Volumen handwarmen Wassers rekonstituiert. Nach 90 Sekunden waren die Klysmen applikationsfähig. Der pH-Wert des rekonstituierten Trockenklysmas lag im physiologisch akzeptablen Bereich von 5,5 - 6,5. Die Löslichkeit der erstarrten Schmelze wurde mikroskopisch bei 400-facher Vergrößerung auf Rekristallisation kontrolliert. Im Kontakt mit Wasser löste sich die erstarrte Schmelze sofort, ohne daß im Beobachtungszeitraum Kristalle sichtbar wurden, ein Beweis dafür, daß sich der Wirkstoff im Zustand einer Feststoffdispersion befand.

### Beispiel 3

730 g 5-Aminosalicylsäure werden mit 10,0 g Natriumlaurylsulfat (Texapon^{(R)}L 100) und 37,0 g wasserfreiem Silicon-Antischaummittel verrieben. In einem Mischer werden 183,0 g Hydroxypropylmethylcellulose (Methocel^{(R)} K15M) und 40,0 g hochdisperse Kieselsäure homogen vermischt. Die Mischung wird über eine Stiftmühle fein vermahlen und auf eine Korngröße < 500 µ abgesiebt. 5,48 g der Trockenklysmenzubereitung entsprechend 4,0 g 5-Aminosalicylsäure, werden in eine graduierte Klysmenflasche gefüllt und mit 50 bzw.100 ml handwarmen Wasser versetzt. Durch Schütteln ist die Rekonstituion nach 2 Minuten abgeschlossen. Die hochviskose Suspension ist weitgehend schaumfrei und erlaubt durch Nachquellung des Gelbildners eine gute Haftfähigkeit an der Darmwand.

### Beispiel 4

752 g 5-Aminosalicylsäure werden mit einer Lösung aus 48 g Mischpolymerisat aus Vinylpyrrolidon/Vinylacetat (Kollidon^{(R)} VA64) und 200 g destiliertem Wasser granuliert. Das getrocknete und auf < 2000 µ zerkleinerte Granulat wird anschließend im Wirbelbett mit einer Lösung bzw. Emulsion aus 65 g Hydroxypropylmethylcellulose (Emcocel K15M) 0,8 g Natriumlaurylsulfat und 10 g Antischaummittel in 5 kg Wasser besprüht.

Das besprühte Granulat besitzt nach dem Trocknen eine Feuchtigkeit von 0,5 % und wird auf eine Korngröße < 500 µ vermahlen. Die Trockenklysmazubereitung wird in graduierte Klysmenflaschen abgefüllt.

### Beispiel 5

8,468 g Natriumoxipurinolmonohydrat werden mit 14,4 g Natriumdihydrogenphosphatmonohydrat, 7,0 g Hydroxypropylmethylcellulose, 1,1 g wasserfreiem Silicon-Antischaummittel und 0,1 g Natriumlaurylsulfat homogen gemischt. 5,43 g der trockenen Zubereitung werden in eine Klysmenflasche dosiert und auf 100 ml Volumen mit handwarmem Wasser versetzt. Nach kurzem Umschütteln ist die entstandene Suspension nach 2 Minuten fertig rekonstituiert. Der pH-Wert beträgt 6,5. Nach visueller Beobachtung findet im Beobachtungszeitraum von 24 Stunden keine Sedimentation statt.

### Beispiel 6

Analog Bsp. 2 wird eine Trockenklysmazubereitung aus 20 g Paracetamol, 170 g Polyethylenglycol 10.000, 9,5 g Hydroxypropylmethylcellulose und 0,5 g wasserfreiem Silicon-Antischaummittel hergestellt.

3 g der Klysmenzubereitung (entsprechend 0,3 g Paracetamol) werden in eine graduierte Klysmenflasche gegeben und mit 5 ml handwarmen Wasser versetzt. Die Rekonstitution ist nach 3 Minuten abgeschlossen. Der gelöste Wirkstoff steht zur raschen Resorption zur Verfügung.

### Beispiel 7

Trockenklysmazubereitungen gemäß Bsp. 2 werden in Klysmenflaschen abgefüllt und auf ihre Langzeitstabilität geprüft. Nach 10-monatiger Lagerung bei Raumtemperatur war keine signifikante Anderung des Wirkstoffgehaltes gegenüber dem Ausgangswert hinsichtlich Gehalt und Zersetzung feststellbar.

### Beispiel 8 (Vergleich)

Eine Suspension bestehend aus:
0,006 g Beclometasondipropionat
24,000 g Propylenglykol
2,160 g p-Hydroxybenzoesäuremethylester
0,240 g p-Hydroxybenzoesäurepropylester
12,000 g Hydroxypropylmethylcellulose
2,55 g Kaliumhydrogenphosphat
ad. 1,20 kg steriles Wasser
wurde mit 2 M Natriumhydroxidlösung auf pH = 5,5 eingestellt.

Es entstand eine fast klare Lösung, die in PE-Klysmenflaschen mit Schraubverschluß zu 100 g abgefüllt wurde. Eine Nachuntersuchung nach 24 Monaten ergab, daß der ursprüngliche Wirkstoffgehalt pro Flasche von 0,5 mg auf 0,28 mg abgesunken war.

Demgegenüber war die erfindungsgemäße Zubereitung gemäß Bsp. 7 nach 20 Monaten unter gleichen Lagerbedingungen stabil.

### Beispiel 9

Ein 59jähriger Mann, mit Morbus Crohn des Colon ascendens und descendens und starken Begleiterkrankungen, wurde mit Mesalazin-Fertigklysmen bzw. Hydrocortisonacetatschaum ohne Erfolg behandelt. Die Behandlung wurde mit Beclometasonklysmen gemäß Beispiel 2 fortgesetzt. Nach wenigen Wochen war die Stuhlfrequenz pro Tag um mehr als die Hälfte reduziert, die Remission hielt bis zum Berichtszeitpunkt bereits 22 Wochen an.

Die Beispiele 8 und 9 zeigen, daß Klysmen der erfindungsgemäßen Art nicht nur gegenüber konventionellen Fertigklysmen außerordentlich stabil sind, sondern auch überraschend wirksam.

## Patentansprüche

1. Pharmazeutische Zubereitung zur rektalen Verabreichung, die in flüssiger Form als wäßrige Lösung, Emulsion oder Suspension eines Wirkstoffs oder Wirkstoffgemischs verabreicht wird, dadurch **gekennzeichnet,** daß sie eine feste Zubereitung darstellt, die 0,01 bis 95 Gew.-% eines Wirkstoffs oder Wirkstoffgemischs sowie 99,99 bis 5 Gew.-% Hilfsstoffe enthält, die 2,5 bis 50 Gew.-% eines hydrophilen Gelbildners, 0,1 bis 10 Gew.-% eines wasserfreien Antischaummittels, von 0 bis 90 Gew.-% wasserlösliche Polymere oder Copolymere oder feste Polyethylenglykole mit einem Molekulargewicht bis zu 35 000 sowie gegebenenfalls weitere Hilfsstoffe umfassen, wobei die Angaben in Gew.-% sich stets auf das Gesamtgewicht der festen Zubereitung beziehen, und daß sie vor der Anwendung in bis zu 10 min durch Zugabe von 5 ml oder mehr Wasser von Raum- oder Körpertemperatur in eine wäßrige Lösung, Emulsion oder Suspension überführbar ist.

2. Zubereitung nach Anspruch 1, dadurch gekennzeichnet, daß sie in Form einer erstarrten Schmelze, eines Granulats oder eines Pulvers vorliegt.

3. Zubereitung nach Anspruch 2, dadurch gekennzeichnet, daß sie in Form eines Pulvers mit einer Teilchengröße von weniger als 1 mm vorliegt.

4. Zubereitung nach einem der Ansprüche 1 bis 3, dadurch gekennzeichnet, daß sie als weitere Hilfsstoffe in Mengen von 0 bis 30 Gew.-% Netzmittel, Fließverbesserer oder Puffersubstanzen zur Einstellung auf isotonische oder physiologische osmotische Verhältnisse enthält.

5. Zubereitung nach Anspruch 1, dadurch gekennzeichnet, daß sie als wasserlösliche Polymere oder Copolymere solche von Vinylpyrrolidon, Vinylacetat oder Vinylalkohol enthält.

6. Zubereitung nach einem der Ansprüche 1 bis 5, dadurch gekennzeichnet, daß sie als Hilfsstoffe zusätzlich feste Verdünnungsmittel und/oder schwerflüchtige flüssige Bestandteile enthält.

7. Zubereitung nach einem der Ansprüche 1 bis 6, dadurch gekennzeichnet, daß sie einen Feuchtigkeitsgehalt von weniger als 15 Gew-%, bezogen auf das Gesamtgewicht der festen Zubereitung, enthält.

8. Zubereitung nach Anspruch 7, dadurch gekennzeichnet, daß der Feuchtigkeitsgehalt weniger als 2 Gew.-% beträgt.

9. Zubereitung nach einem der Ansprüche 1 bis 8, dadurch gekennzeichnet, daß sie frei von Konservantien und Stabilisatoren ist.

10. Zubereitung nach einem der Ansprüche 1 bis 9, dadurch gekennzeichnet, daß sie sich in einer Klysmenflasche mit Graduierung befindet.

11. Zubereitung nach einem der Ansprüche 1 bis 10, dadurch gekennzeichnet, daß sie mindestens einen der Wirkstoffe Beclomethason, Betamethason, 5-Aminosalicylsäure, Oxipurinol oder Sulfasalazin oder ein Salz, einen Ester oder ein Hydrat oder ein anderes Derivat davon enthält.

## Claims

1. A pharmaceutical preparation for rectal administration, which is administered in liquid form as an aqueous solution, emulsion or suspension of an active ingredient or a mixture of active ingredients, characterized in that it is a solid preparation containing 0.01 to 95% by weight of an active ingredient or a mixture of active ingredients and also 99.99 to 5% by weight of adjuvants which comprise 2.5 to 50% by weight of a hydrophilic gel former, 0.1 to 10% by weight of an anhydrous anti-foaming agent, from 0 to 90% by weight of water-soluble polymers or copolymers or solid polyethylene glycols having a molecular weight of up to 35 000 and also if necessary other adjuvants, the statements of percentages by weight always referring to the total weight of the solid preparation, and prior to use the preparation can be converted into an aqueous solution, emulsion or suspension in up to 10 minutes by the addition of 5 ml or more of water at room or body temperature.

2. A preparation according to claim 1, characterized in that it takes the form of a solidified melt, a granulate or a powder.

3. A preparation according to claim 2, characterized in that it takes the form of a powder having a particle size of less than 1 mm.

4. A preparation according to one of claims 1 to 3, characterized in that it contains as further adjuvants in quantities of 0 to 30% by weight wetting agents, flow-improving agents or buffer substances for adjustment to isotonic or physiological osmotic conditions.

5. A preparation according to claim 1, characterized in that it contains vinyl pyrrolidone, vinyl acetate or vinyl alcohol as water-soluble polymers or copolymers.

6. A preparation according to claims 1 to 5, characterized in that it also contains as adjuvants solid diluents and/or difficultly volatile liquid components.

7. A preparation according to claims 1 to 6, characterized in that it contains a moisture content of less than 15% by weight, referred to the total weight of the solid preparation.

8. A preparation according to claim 7, characterized in that the moisture content is lower than 2%.

9. A preparation according to one of claims 1 to 8, characterized in that it is free from preservatives and stabilizers.

10. A preparation according to one of claims 1 to 9, characterized in that it is contained in a graduated enema flask.

11. A preparation according to one of claims 1 to 10, characterized in that it contains at least one of the following ingredients: beclomethasone, betamethasone, 5-aminosalicylic acid, oxipurinol or sulphasalazine or a salt, an ester or a hydrate or other derivative thereof.

## Revendications

1. Préparation pharmaceutique destinée à l'administration par voie rectale, que l'on administre sous la forme liquide comme solution, émulsion ou suspension aqueuse d'une substance active ou d'un mélange de substances actives, caractérisée en ce qu'elle représente une préparation solide qui contient 0,01 à 95 % en poids d'une substance active ou d'un mélange de substances actives ainsi que 99,99 à 5 % en poids de substances auxiliaires qui comprennent 2,5 à 50 % en poids d'un gélifiant hydrophile, 0,1 à 10 % en poids d'un agent anti-moussant anhydre, 0 à 90 % en poids de polymères ou de copolymères hydrosolubles ou de polyéthylène-glycols solides de poids moléculaire allant jusqu'à 35 000 ainsi que, le cas échéant, d'autres substances auxiliaires, les indications de pourcentages en poids se rapportant toujours au poids total de la préparation solide, et en ce qu'elle peut être transformée en une solution, émulsion ou suspension aqueuse en un laps de temps allant jusqu'à 10 minutes avant l'utilisation par addition de 5 ml ou plus de 5 ml d'eau à la température ambiante ou à la température du corps.

2. Préparation suivant la revendication 1, caractérisée en ce qu'elle est sous la forme d'une masse fondue solidifiée, de granules ou d'une poudre.

3. Préparation suivant la revendication 2, caractérisée en ce qu'elle se présente sous la forme d'une poudre en particules de diamètre inférieur à 1 mm.

4. Préparation suivant l'une des revendications 1 à 3, caractérisée en ce qu'elle contient comme autres substances auxiliaires, en quantités de 0 à 30 % en poids, des agents mouillants, des agents améliorant l'écoulement ou des substances tampons permettant d'établir des conditions isotoniques ou osmotiques du point de vue physiologique.

5. Préparation suivant la revendication 1, caractérisée en ce qu'elle contient comme polymères ou copolymères hydrosolubles des polymères ou copolymères de vinylpyrrolidone, d'acétate de vinyle ou d'alcool vinylique.

6. Préparation suivant l'une des revendications 1 à 5, caractérisée en ce qu'elle contient en outre comme substances auxiliaires des diluants solides et/ou des constituants liquides peu volatils.

7. Préparation suivant l'une des revendications 1 à 6, caractérisée en ce qu'elle présente une teneur en humidité de moins de 15 % en poids par rapport au poids total de la préparation solide.

8. Préparation suivant la revendication 7, caractérisée en ce que la teneur en humidité est inférieure à 2 % en poids.

9. Préparation suivant l'une des revendications 1 à 8, caractérisée en ce qu'elle est dépourvue d'agents de conservation et d'agents stabilisants.

10. Préparation suivant l'une des revendications 1 à 9, caractérisée en ce qu'elle se trouve dans un flacon pour lavement portant une graduation.

11. Préparation suivant l'une des revendications 1 à 10, caractérisée en ce qu'elle contient au moins l'une des substances actives béclométasone, bêtamétasone, acide 5-aminosalicylique, oxypurinol ou sulfasalazine ou un sel, un ester ou un hydrate ou un autre dérivé de cette substance.
